# EUROPÄISCHE PATENTSCHRIFT

(11) **EP 2 790 602 B1**
(45) Veröffentlichungstag und Bekanntmachung des Hinweises auf die Patenterteilung: **08.07.2015**
(21) Anmeldenummer: 12783893.6
(22) Anmeldetag: 30.10.2012
(51) Int. Cl.: A61B 17/00, A61B 18/00, A61B 18/12, A61B 19/00, A61B 18/14

(54) **RESEKTOSKOP MIT EINEM SCHAFT**
RESECTOSCOPE COMPRISING A SHAFT
RÉSECTOSCOPE À TIGE

(30) Priorität: 15.12.2011 DE 102011121159
(43) Veröffentlichungstag der Anmeldung: 22.10.2014
(73) Patentinhaber: Olympus Winter & Ibe GmbH, 22045 Hamburg (DE)
(72) Erfinder: HIRSCHFELD, Simon, 22393 Hamburg (DE)
(74) Vertreter: Hausfeld, Norbert
(86) Internationale Anmeldenummer: PCT/EP2012/004529
(87) Internationale Veröffentlichungsnummer: WO 2013/087132

(56) Entgegenhaltungen:
- US-A- 4 744 361
- US-A- 4 917 621
- US-A- 5 919 191
- US-A1- 2004 044 343

## Beschreibung

Die Erfindung betrifft ein Resektoskop der im Oberbegriff des Anspruches 1 genannten Art.

Ein gattungsgemäßes Resektoskop ist in der DE 10 2004 045 337 A1 dargestellt. Dabei ist der Schlittenkontakt als Steckbuchse und der Elektrodenkontakt als Steckstift ausgebildet. Diese Konstruktionsweise entspricht der bisher stets verfolgten Konstruktionsrichtung, die Wert darauf legte, die Elektrodenanordnung als Einmalartikel so einfach und kostengünstig wie möglich zu gestalten und alle aufwändigeren Konstruktionselemente am wiederverwendbaren Schlitten anzuordnen.

Ein Steckstift ist konstruktiv einfacher und daher kostengünstiger gegenüber einer Steckbuchse, die hohl und beispielsweise mit einer Schlitzung federnd ausgebildet sein kann.

Eine weitere gattungsgemäße Konstruktion ist in der DE 20 2005 013 853 U1 dargestellt. Hier sieht man, dass in Weiterverfolgung der erwähnten Konstruktionsrichtung auch ein abdichtender O-Ring am Schlitten und nicht an der Elektrodenanordnung ausgebildet ist, ebenso wie eine Rasteinrichtung, die ebenfalls schlittenseitig angeordnet ist.

Diese bisher verfolgte konstruktive Richtung führt jedoch auch zu Nachteilen. Der Schlitten ist ein konstruktiv aufwändiges und teures Konstruktionselement, das daher üblicherweise wiederverwendbar ausgebildet wird. Das bedeutet, dass er nach jeder Benutzung gereinigt und sterilisiert werden muss. Bei einer in der Aufnahmebohrung des Schlittens angeordneten Steckbuchse ist die Reinigung aber sehr schwierig. Weiterhin von Nachteil ist die bei den gegebenen sehr kleinen Abmessungen außerordentlich dünnwandige und fragile Konstruktion der Steckbuchse, die beim Einstecken des Steckstiftes leicht verbiegen kann und dann eine teure Reparatur am Schlitten erfordert, wozu das Resektoskop in eine Werkstatt geschickt werden muss. Die Kontakte können auch überhitzen, wenn z. B. aufgrund von Verschmutzung ein erhöhter Kontaktwiderstand vorliegt. Bei den zu übertragenden Hochfrequenzströmen kann dies sehr schnell zur Zerstörung führen. Auch dann sind teure Reparaturen am Schlitten erforderlich.

Die Aufgabe der vorliegenden Erfindung besteht darin, die erwähnten Reinigungs- und Reparaturprobleme zu vermeiden.

Diese Aufgabe wird erfindungsgemäß mit den Merkmalen des Kennzeichnungsteils des Anspruches 1 gelöst.

Erfindungsgemäß ist der Schlittenkontakt als Steckstift und der Elektrodenkontakt folglich als Steckbuchse ausgebildet. Es wird also die Philosophie der möglichst einfachen Elektrodenanordnung aufgegeben und unter radikalem Umdenken eine kompliziertere und teurere Bauweise der Elektrodenanordnung zugelassen, an der nun die Steckbuchse erforderlich ist. Das führt zwar zu einer teureren Elektrodenanordnung aber dafür zu erheblichen Verbesserungen am Schlitten. Am Schlitten sitzt nunmehr der Steckstift. Dieser ist wesentlich einfacher zu reinigen und ist von Überwärmung und elektrischen Abbrand aufgrund seiner einfacheren Konstruktion weniger gefährdet. Er ist auch mechanisch robuster und wird beim Einstecken weniger leicht beschädigt. Die in allen diesen Punkten anfälligere Steckbuchse sitzt erfindungsgemäß an der Elektrodenanordnung. Dort stört die schlechte Reinigbarkeit nicht, da die Elektrodenanordnung ein Einmalartikel ist und nicht gereinigt wird. Die mechanische und elektrische Anfälligkeit der Steckbuchse stört bei der Elektrodenanordnung auch nur wenig, da die Steckbuchse mit der Elektrodenanordnung ohnehin nach jeder Benutzung erneuert wird. Der erfindungsgemäß ausgebildete Schlitten hat eine wesentlich verringerte Störanfälligkeit und eine wesentlich verbesserte Reinigbarkeit. Insgesamt ergibt sind sowohl auf Seiten der Elektrodenanordnung als auch auf Seiten des Schlittens eine Verringerung der Störanfälligkeit und dadurch auch der Reparatur- und Wartungskosten.

Die Erfindung lässt sich auch bei einer bipolaren Elektrodenanordnung verwenden. Es könnten z. B. an der Elektrodenanordnung zwei Steckbuchsen parallel angeordnet sein. Vorteilhaft gemäß Anspruch 2 ist jedoch der zweite Elektrodenkontakt ringförmig angeordnet, woraus sich eine robustere und rotationssymmetrische Konstruktion ergibt.

Die zweite Elektrodenanordnung erfordert auf einer der beiden zu kontaktierenden Seiten federnde Elemente. Vorteilhaft gemäß Anspruch 3 sind dazu am Umfang der Elektrodenanordnung radial federnde Zungen ausgebildet, wobei wiederum das aufwändigere und störanfälligere Element an der Elektrodenanordnung ausgebildet ist.

Vorteilhaft gemäß Anspruch 4 ist auch eine Ringdichtung an der Elektrodenanordnung ausgebildet und gemäß Anspruch 5 eine federnde Raste ebenfalls an der Elektrodenanordnung.

Nach Anspruch 6 ist die erfindungsgemässe Elektrodenanordnung und nach Anspruch 7 der erfindungsgemässe Schlitten unter Schutz gestellt.

In der Zeichnung ist die Erfindung beispielsweise und schematisch dargestellt. Es zeigen:
- Fig. 1: die Seitenansicht eines erfindungsgemäßen Resektoskopes,
- Fig. 2: in vergrößertem Längsschnitt den Schlitten der Konstruktion der Fig. 1 und
- Fig. 3: in vergrößertem Längsschnitt, wobei die Dicke stärker vergrößert ist als die Länge, den proximalen Endbereich der Elektrodenanordnung des Resektoskopes der Fig. 1.

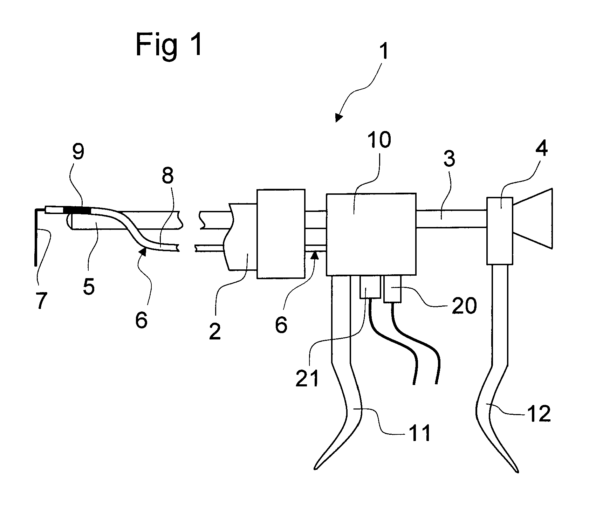

Fig. 1 zeigt in Seitenansicht ein Resektoskop 1 mit einem Schaft 2, der an seinem proximalen Ende über ein Optikführungsrohr 3 mit einer Abschlussplatte 4 verbunden ist. Durch diese Abschlussplatte 4 und das Optikführungsrohr 3 lässt sich bis in den Schaft 2 hinein eine Optik 5 einschieben, von deren distalem Ende aus das mit dem Resektoskop 1 zu bearbeitende Operationsgebiet eingesehen werden kann.

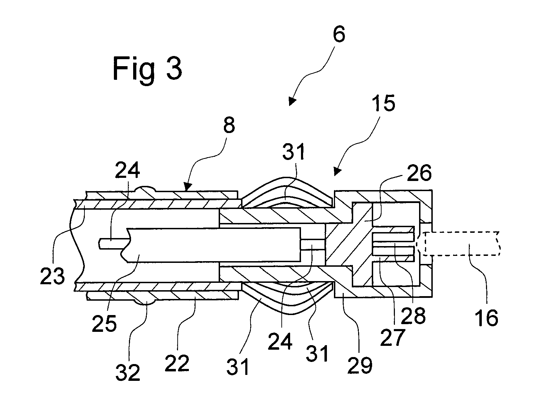

Im Schaft 2 ist ferner eine Elektrodenanordnung 6 in Längsrichtung des Schaftes 2 verschiebbar angeordnet. Am distalen Ende der Elektrodenanordnung 6 ist eine Schneidschlinge 7 in einer für Resektoskope typischen Ausbildung angeordnet. Die Schneidschlinge 7 wird von einem Elektrodenträger 8 gehalten, der z. B. im distalen Endbereich in der üblichen Gabelform ausgebildet ist. Der Elektrodenträger 8 wird in der dargestellten Ausführungsform von einem elektrischen Leiter 24 (Fig. 3) durchlaufen, der nach außen isoliert ist und den Stromanschluss für die mit Hochfrequenz zu beaufschlagende Schneidschlinge 7 ausbildet.

Im distalen Endbereich des Elektrodenträgers 8 ist auf diesem eine Rückleit- oder Returnelektrode 9 als nach außen freiliegender Bereich ausgebildet. Auch diese ist über einen die Elektrodenanordnung durchlaufenden weiteren Leiter kontaktiert. Die dargestellte Elektrodenanordnung 6 kann in monopolarer Ausbildung auch ohne die Returnelektrode 9 ausgebildet sein. Ferner kann die Returnelektrode auch in anderer Form und an anderer Stelle angeordnet sein. So kann z.B. der Schaft 2 die Returnelektrode ausbilden.

Das Resektoskop 1 weist einen Schlitten 10 auf, der in Richtung des Schaftes 2 verschiebbar gelagert ist. In üblicher, im Ausführungsbeispiel verwendeter Konstruktion ist dabei der Schlitten 10 auf dem Optikführungsrohr 3 längsverschiebbar gelagert. Mit Griffstücken 11 und 12 am Schlitten 10, bzw. an der Abschlussplatte 4 lässt sich der Schlitten 10 in proximaler Richtung bewegen, wobei die Schneidschlinge 7 die typische zurückziehende Bewegung ausführt, mit der der Arzt unter Hochfrequenzbeaufschlagung der Schneidschlinge gewebeabtragende Schnitte z.B. in der Prostata ausführt.

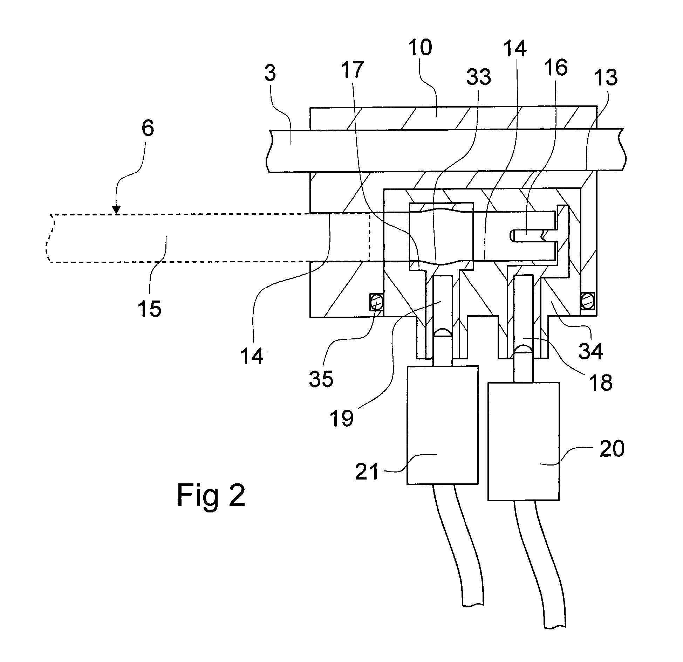

Fig. 2 zeigt im Längsschnitt die innere Konstruktion des Schlittens 10, der mit einer Führungsbohrung 13 auf dem Optikführungsrohr 3 geführt ist. Parallel zur Führungsbohrung 13 ist im Schlitten 10 eine Aufnahmebohrung 14 ausgebildet, in die der in Fig. 2 gestrichelt dargestellte proximale Endbereich 15 der Elektrodenanordnung 6 einsteckbar ist.

Am proximalen Ende der Aufnahmebohrung 14 ragt ein Steckstift 16 in axialer Richtung in die Aufnahmebohrung 14 hinein. Distal beabstandet zum Steckstift 16 ist in der Innenwand der Aufnahmebohrung 14 ein Ringkontakt 17 angeordnet. Der Steckstift 16 steht parallel zur Achse der Aufnahmebohrung 14 und kann sich zum Erleichtern des Einsteckens in distaler Richtung verjüngen.

Der Steckstift 16 ist elektrisch mit einer Buchse 18 verbunden und zwar im in Fig. 2 gezeigten Ausführungsbeispiel einstückig. Der Ringkontakt 17 ist mit einer weiteren Buchse 19 elektrisch verbunden. In die Buchsen 18 und 19 können, wie in Fig. 2 dargestellt, Stecker 20 und 21 eingesteckt werden, die über Kabel an einen Hochfrequenzgenerator verbinden. Auf diese Weise werden die beiden Pole eines Hochfrequenzgenerators an die Elektroden 7 und 9 der Elektrodenanordnung 6 verbunden.

Zur Kontaktierung der in Fig. 2 dargestellten Kontaktanordnung im Schlitten 10 ist der proximale Endbereich 15 der Elektrodenanordnung 6 so ausgebildet, wie dies die Fig. 3 im Längsschnitt zeigt, bei dem die Dicke stärker vergrössert ist als die Länge.

Der Elektrodenträger 8 ist, wie bereits erwähnt, außen isoliert, und zwar mit einer Isolierhülle 22. Diese umschließt ein aus Metall bestehendes, elektrisch leitendes Innenrohr 23, das den Leiter bildet, der die Returnelektrode 9 versorgt. Am Ort der Returnelektrode 9 kann das Innenrohr 23 selbst die Elektrode ausbilden, indem an dieser Stelle die Isolierhülle 22 weggelassen ist.

Der die Schneidschlinge 7 kontaktierender Leiter 24 ist innerhalb des Innenrohres 23 angeordnet und diesem gegenüber mit einem Isoliermantel 25 isoliert.

Das proximale Ende des Leiters 24 ist mit einem Kontaktstück 26 kontaktiert, beispielsweise durch Verlötung. Das Kontaktstück 26 besteht aus Metall und trägt eine Steckbuchse 27, die sich vom Kontaktstück 26 aus in proximaler Richtung erstreckt und die über wenigstens einen in axialer Richtung erstreckten Schlitz 28 verfügt, der dafür sorgt, dass die Steckbuchse 27 radial federn kann. Die Steckbuchse 27 bildet das proximale Ende der Elektrodenanordnung 6 aus. Ihr Innendurchmesser kann sich in proximaler Richtung vergrössernd ausgebildet sein, um den Aufsteckvorgang zu erleichtern.

Fig. 3 zeigt gestrichelt den Steckstift 16, noch außerhalb der Steckbuchse 27 stehend. Wird die Elektrodenanordnung 6 weiter in proximaler Richtung geschoben, so wird die Steckbuchse 27 auf den Steckstift 16 aufgesteckt und kontaktiert diesen klemmend.

Das Kontaktstück 26 ist im Inneren einer Isolierkappe 29 angeordnet, die mit einem zentralen Loch den Steckstift 16 passieren lässt und die das Kontaktstück 26 und die Steckbuchse 27 nach außen isolierend abschirmt. Die Isolierkappe 29 reicht so weit in distale Richtung, dass sie auch den freiliegenden proximalen Endbereich des Leiters 24 isolierend umgibt. Im Ausführungsbeispiel greift sie bis in den proximalen Endbereich des elektrisch leitenden Innenrohres 23 und schafft eine mechanische Verbindung zu diesem sowie eine durchgehende Isolierung.

Am proximalen Ende des elektrisch leitenden Innenrohres 23 sind als zweiter Elektrodenkontakt Federzungen 31 angeordnet, die z. B. durch Längsschlitzung des Innenrohres 23 in seinem proximalen Endbereich ausgebildet sein können. Die Federzungen 31 sind nach außen gewölbt und federnd ausgebildet. Beim Einstecken des Endbereiches 15 der Fig. 3 in die Aufnahmebohrung 14 des in Fig. 2 dargestellten Schlittens 10 bis in die Endstellung wird somit die Steckbuchse 27 auf den Stift 16 gesteckt und es kommen die Federzungen 31 in federnder Anlage an die Innenfläche des Ringkontaktes 17. In eingesteckter Stellung ist folglich die Schneidschlinge 7 der Elektrodenanordnung 6 mit dem Stecker 20 und die Returnelektrode 9 mit dem Stecker 21 verbunden.

Durch die Isolierkappe 29 wird der Isolierabstand zwischen den beiden proximalen Endkontakten des Elektrodenträgers 6, nämlich zwischen der Steckbuchse 27 und den Federzungen 31 vergrössert und störender Stromfluss zwischen diesen verhindert.

Im Ausführungsbeispiel ist ein Dichtring vorgesehen, der bei in die Aufnahmebohrung 14 eingestecktem Endbereich 15 für Abdichtung sorgt, damit keine Flüssigkeit in das Innere der Aufnahmebohrung 14 eindringen kann. Dazu ist ein den Endbereich 15 radial nach außen überragender elastischer Dichtring vorgesehen, der im dargestellten Ausführungsbeispiel der Fig. 3 als Ringwulst 32 ausgebildet ist, der einstückig mit der Isolierhülle 22 ausgebildet ist. Der Dichtring kann auch in anderer Ausbildung und an anderer Stelle vorgesehen sein. Er kann z.B. als O-Ring in einer Umfangsnut gelagert sein, die an der Stelle der Ringwulst 32 in der Isolierhülle 22 ausgebildet ist

Im dargestellten Ausführungsbeispiel weist der Ringkontakt 17, wie Fig. 2 zeigt, eine Innenfläche auf, die in der Mitte gegenüber den Enden etwas erweitert ist. Diese Umfangserweiterung bildet eine flache Ringnut 33 aus, in die die Federzungen 31 einrasten können, unter Ausbildung eines federnden Rasteingriffes, der die Endposition der Einsteckbewegung sichert und für Aufrechterhaltung des gewünschten Kontaktzustandes sorgt. Das Erreichen des korrekten Rasteingriffes kann dem Bediener angezeigt werden, z.B. durch ein Einrastgeräusch oder durch eine Bewegung, die z.B. gut sichtbar oder mit der Hand fühlbar ist.

In der dargestellten Ausführungsform des Schlittens 10 ist dieser, wie der eingangs genannte Stand der Technik zeigt, mit einem auswechselbaren Einsatz 34 versehen, mit dem der Steckstift 16 und der Ringkontakt 17 gewechselt werden können. Ein den Einsatz 34 umlaufender O-Ring 35 sorgt dabei für Abdichtung.

Der Elektrodenträger 6 kann anstelle der in den Figuren gezeigten Schneidschlinge 7 auch eine anders geformte Elektrode tragen, z.B. eine flächige Vaporisationselektrode.

Zur Vermittlung einer Grössenvorstellung der vorstehend beschriebenen Konstruktion wird erwähnt, dass der Aussendurchmesser des proximalen Endbereiches 15 der Elektrodenanordnung 6 bevorzugt in dem aus dem Stand der Technik für solche Elektrodenanordnungen bekannten Grössenbereich von ca. 1,5 bis 2 mm liegt.

## Patentansprüche

1. Resektoskop (1) mit einem Schaft (2) und einer in Richtung des Schaftes (2) verschiebbar gelagerten Elektrodenanordnung (6) und einem in derselben Richtung verschiebbaren Schlitten (10), der mit einem Schlittenkontakt (16) und einer parallel zur Schaftrichtung erstreckten Aufnahmebohrung (14) ausgebildet ist, in welche der proximale Endbereich (15) der Elektrodenanordnung (6) mit einem Elektrodenkontakt (27) zur Kontaktierung des Schlittenkontaktes (16) einsteckbar ist, wobei einer der Kontakte (16, 27) als Steckbuchse und der andere als dazu passender Steckstift ausgebildet ist, **dadurch gekennzeichnet, dass** der Schlittenkontakt als Steckstift (16) ausgebildet ist.

2. Resektoskop nach Anspruch 1, mit bipolar ausgebildeter Elektrodenanordnung (6) und zwei Elektrodenkontakten (27, 31) zur Kontaktierung zweier Schlittenkontakte (16, 17), **dadurch gekennzeichnet, dass** die Steckbuchse (27) am proximalen Ende der Elektrodenanordnung (6) angeordnet ist und dass ein zweiter Elektrodenkontakt (31) auf der Außenseite des zylindrisch ausgebildeten Endbereiches (15) der Elektrodenanordnung (6) angeordnet ist.

3. Resektoskop nach Anspruch 2, **dadurch gekennzeichnet, dass** der zweite Elektrodenkontakt am Umfang der Elektrodenanordnung (6) ausgebildete, radial federnde Federzungen (31) aufweist.

4. Resektoskop nach einem der vorhergehenden Ansprüche, **dadurch gekennzeichnet, dass** am Endbereich (15) der Elektrodenanordnung (6) eine Ringdichtung (32) zum Abdichten der Aufnahmebohrung (14) angeordnet ist.

5. Resektoskop nach einem der vorherigen Ansprüche, **dadurch gekennzeichnet, dass** am Endbereich (15) der Elektrodenanordnung (6) eine federnde Raste (31) zum Einrasten in eine Vertiefung (33) in der Innenfläche der Aufnahmebohrung (14) angeordnet ist.

6. Elektrodenanordnung (6) zur Verwendung in einem Resektoskop (1) nach einem der vorhergehenden Ansprüche, mit einer Elektrode (7) am distalen Ende und einem an diese angeschlossenen Elektrodenkontakt (27) am proximalen Ende, **dadurch gekennzeichnet, dass** der Elektrodenkontakt als Steckbuchse (27) ausgebildet ist.

7. Schlitten (10) zur Verwendung in einem Resektoskop (1) nach einem der Ansprüche 1 bis 5, mit einer Aufnahmebohrung (14), in die eine Elektrodenanordnung (6) einsteckbar ist, und mit einem am proximalen Ende der Aufnahmebohrung (14) angeordneten Schlittenkontakt(16), der von einem Elektrodenkontakt (27) der Elektrodenanordnung (69 kontaktierbar ist, **dadurch gekennzeichnet, dass** der Schlittenkontakt als Steckstift (16) ausgebildet ist.

## Claims

1. A resectoscope (1) having a shank (2) and an electrode arrangement (6) that is mounted such that it can be moved in the direction of the shank (2) and a carriage (10) that can be moved in the same direction, said carriage (10) being configured with a carriage contact (16) and a receiving hole (14) extending parallel to the shank direction, wherein the proximal end region (15) of the electrode arrangement (6) can be inserted into said receiving hole (14) with an electrode contact (27) for contacting the carriage contact (16), wherein one of the contacts (16, 27) is configured as a socket and the other one is configured as a pin mating therewith, **characterised in that** the carriage contact is configured as a pin (16).

2. The resectoscope according to Claim 1, having a bipolar electrode arrangement (6) and two electrode contacts (27, 31) for contacting two carriage contacts (16, 17), **characterised in that** the socket (27) is arranged at the proximal end of the electrode arrangement (6) and that a second electrode contact (31) is arranged on the outside of the cylindrical end region (15) of the electrode arrangement (6).

3. The resectoscope according to Claim 2, **characterised in that** the second electrode contact includes radially resilient flexible tongues (31) that are formed along the circumference of the electrode arrangement (6).

4. The resectoscope according to any one of the preceding claims, **characterised in that** a ring seal (32) for sealing the receiving hole (14) is arranged at the end region (15) of the electrode arrangement (6).

5. The resectoscope according to any one of the preceding claims, **characterised in that** a resilient pawl (31) for being locked in place in a recess (33) in the inner surface of the receiving hole (14) is arranged at the end region (15) of the electrode arrangement (6).

6. An electrode arrangement (6) for use in a resectoscope (1) according to any one of the preceding claims, having an electrode (7) at its distal end and an electrode contact (27) at its proximal end, said electrode contact (27) being connected to said electrode (7), **characterised in that** the electrode contact is configured as a socket (27).

7. A carriage (10) for use in a resectoscope (1) according to any one of Claims 1 to 5, having a receiving hole (14) into which an electrode arrangement (6) can be inserted, and having a carriage contact (16) that is arranged at the proximal end of the receiving hole (14) and can be contacted by an electrode contact (27) of the electrode arrangement (6), **characterised in that** the carriage contact is configured as a pin (16).

## Revendications

1. Résectoscope (1) avec une gaine (2) et un dispositif à électrode (6) logé de façon à pouvoir être déplacé dans la direction de la gaine (2) et un coulisseau (10) également déplaçable dans le même sens et équipé d'un contact (16) du coulisseau et muni d'un alésage (14) s'étendant parallèlement à l'axe de la gaine et formant un logement dans lequel la section d'extrémité proximale (15) du dispositif à électrode (6) est enfichable avec un contact d'électrode (27) établissant le contact avec le contact du coulisseau (16), l'un des contacts (16, 27) étant conçu sous forme de douille et l'autre sous forme de fiche correspondante, **caractérisé en ce que** le contact du coulisseau est réalisé sous forme de fiche (16).

2. Résectoscope selon la revendication 1 avec un dispositif à électrode (6) de construction bipolaire et deux contacts d'électrode (27, 31) établissant le contact avec deux contacts du coulisseau (16, 17), **caractérisé en ce que** la douille (27) est disposée à l'extrémité proximale du dispositif à électrode (6) et qu'un second contact d'électrode (31) est disposé sur la face externe de la section d'extrémité de conformation cylindrique (15) du dispositif à électrode (6).

3. Résectoscope selon la revendication 2, **caractérisé en ce que** le second contact d'électrode présente des languettes (31) faisant ressort en sens radial formées à la périphérie du dispositif à électrode (6).

4. Résectoscope selon l'une des revendications précédentes, **caractérisé en ce qu'**un joint annulaire (32) est placé à la section d'extrémité (15) du dispositif à électrode (6) pour étancher l'alésage (14).

5. Résectoscope selon l'une des revendications précédentes, **caractérisé en ce qu'**un cliquet (31) faisant ressort est placé à la section d'extrémité (15) du dispositif à électrode (6) pour s'enclencher dans une concavité (33) réservée sur la face intérieure de l'alésage (14).

6. Dispositif à électrode (6) destiné à être utilisé avec un résectoscope (1) selon l'une des revendications précédentes avec, à son extrémité distale, une électrode (7) et, à son extrémité proximale, un contact d'électrode (27) connecté à ladite électrode, **caractérisé en ce que** le contact d'électrode est réalisé sous forme de douille (27).

7. Coulisseau (10) destiné à être utilisé avec un résectoscope (1) selon l'une des revendications 1 à 5, avec un alésage (14) dans lequel peut être enfiché un dispositif à électrode (6), et avec un contact (16) disposé à l'extrémité proximale de l'alésage (14), ledit contact pouvant être contacté par un contact d'électrode (27) du dispositif à électrode (6), **caractérisé en ce que** le contact du coulisseau est réalisé sous forme de fiche (16).
